# EUROPEAN PATENT APPLICATION

(11) **EP 2 704 112 A1**
(43) Date of publication of application: **05.03.2014**
(21) Application number: 12182648.1
(22) Date of filing: 31.08.2012
(51) Int. Cl.: G07F 11/68, G07F 17/00, B26D 5/00

(54) **Method for operating a medication dispenser, and medication dispenser**

(71) Applicant: Evondos Oy, 24240 Salo (FI)
(72) Inventor: Apell, Mika, 20700 Turku (FI); Niinistö, Jyrki, 24800 Halikko (FI)
(74) Representative: Mäkelä, Katja

(57) **Abstract**

The present invention relates to a method in which the position of a seam between two consecutive packages (103) in a strip (102) is detected. In the method the strip (102) is moved across an imaging area of a camera (105), at least one image of the strip (102) is captured, the at least one image is analysed using pattern recognition to detect the position of the seam, and the steps of moving the strip (102), capturing at least one image and analysing the at least one image are repeated until the position of the seam is detected. The invention also relates to a medication dispenser (100).

## Description

### TECHNICAL FIELD OF THE INVENTION

The present invention relates to a method for operating a medication dispenser and to a medication dispenser according to the preambles of the appended independent claims.

### BACKGROUND OF THE INVENTION

Various devices are known for assisting a patient in complying with his/her medical regimen. The most sophisticated devices are so-called medication dispensers, which dispense prepackaged and labelled medication packages to provide the patient with the proper dosage of medications at a prescribed time. The medications are prepackaged into packages according to the medical regimen of the patient, and are available from licensed pharmacies. The labels of the medication packages may contain information about the patient, the content of the package, and the time of the dosage.

Typically, the medication packages are arranged as a strip, which is inserted into a container of the medication dispenser either by the patient or a caregiver of the patient. The medication dispenser delivers the packages to the patient one package at a time according to the information provided by the labels of the packages, or information stored in the medication dispenser. The medication dispenser allows the dispensation of medications to be monitored and controlled so that the patient, the caregiver or any other person having access to the apparatus can be assured that the patient is taking the medications as prescribed.

A problem associated with the medication dispensers of the prior art relates to the recognition of packages in a strip and separating the packages from the strip. In a known medication dispenser the recognition of packages is based on the recognition of printed information on labels of the packages. Each package has a label that contains a certain marking according to which the medication dispenser identifies the packages of the strip from each other. Based on this information, the medication dispenser determines the positions at which the packages may be separated from the strip. Because the positions of the seams between the consecutive packages are not known, there is a risk of separating the packages at wrong places.

Another problem associated with the medication dispensers of the prior art relates to their dependency of a certain label layout. Known medication dispensers are capable of only recognising information on labels having a specific layout. If a different layout is used in labels, the medication dispenser cannot read the information on the label, and therefore the medications cannot be dispensed.

### OBJECTIVES OF THE INVENTION

It is the main objective of the present invention to reduce or even eliminate prior art problems presented above.

It is an objective of the present invention to provide a method and a medication dispenser enabling to detect the position of the seam between two consecutive packages in a strip of packages containing medications to be taken at predetermined taking times. It is also an objective of the invention to provide a method and a medication dispenser enabling to recognise information on different types of labels.

In order to realise the above-mentioned objectives, the method and the medication dispenser according to the invention are characterised by what is presented in the characterising parts of the appended independent claims. Advantageous embodiments of the invention are described in the dependent claims.

### DESCRIPTION OF THE INVENTION

A typical method according to the invention for operating a medication dispenser that includes a strip of packages containing medications to be taken at predetermined taking times, comprises detecting the position of a seam between two consecutive packages in the strip, the step comprising the substeps of moving the strip across an imaging area of a camera, capturing at least one image of the strip, analysing the at least one image using pattern recognition to detect the position of the seam, and repeating the substeps of moving the strip, capturing at least one image and analysing the at least one image, until the position of the seam is detected.

The method according to the invention concerns recognition of a seam between two consecutive packages in a strip. The method is based on taking one or more images of the strip and analysing the images using pattern recognition. The images can be analysed separately, or the images can be combined to a single image in order to enhance the image quality. Pattern recognition in seam recognition means processing the image data in vertical and/or horizontal directions to find a potential position for the seam. This can be based on detecting unprinted horizontal areas on the strip. A layout of a package label is typically similar in each package of the strip. A label layout consists of information printed in different specific positions in a specific form. A potential position of the seam can be detected between these consecutive label layout patterns. After a potential position of the seam has been found, it is analysed in more detail to find elements that are typical to a seam. A seam typically consists of elements, such as perforation and/or glued areas, which can be distinguished from the other parts of the strip. Perforation and gluing affect the surface and transparency of the package. The surface is flatter outside the perforation and glued areas, and the perforation is recognised as a series of dots. On the other hand, the transparency of a thin strip material is different on the seam area due to glue and perforation. They also differ from other detectable patterns on the package surface, because gluing and perforation is done from one edge of the strip to another edge of the strip.

A package layout may include information about the position of the seam in proportion to a package label, about the seam pattern parameters like the width of the seam area including glued areas and perforation, or only about the width of each glued area. It may also include information about the processing parameters, such as environment parameters including lighting intensity, direction or wavelength, or digital filters and their parameters for each specific strip, to enhance seam recognition. This seam specific information in a package layout can be updated based on the information that has been gathered during the processing to optimise the seam detection accuracy.

The strip can be moved across the imaging area of the camera continuously or discontinuously. The images are preferably taken of the strip when the strip is not moving. Once the strip is moved to a new place, one or more images may be taken of the strip. Images may also be taken while the strip is being moved. The strip may be moved in one direction, or back and forth across the imaging area. Between the capturing of images, imaging conditions may be changed. It is also possible to move the camera in a direction of the width of the strip between the capturing of images.

By the imaging area is meant the area of which the camera is capable of capturing images. The imaging area is preferably rectangular and the aspect ratio is typical to image sensors, for example 16:9 or 4:3. Preferably, the camera is positioned in such a manner that the whole width of the strip fits in the imaging area and thus in a single image. If the strip is wider than the imaging area, the camera can be moved in a direction of the width of the strip and a plurality of images can be captured and then combined.

Different changes for an imaging environment or target can be made between taking images to be combined for enhanced recognition accuracy, for example, camera movements, strip movements and/or lighting adjustments. Several image processing techniques can be utilised for combining, for example, HDR (High Dynamic Range) or stereo imaging techniques.

The position information of the seam is utilised in separating the first package from the second package. Based on the position of the seam a cutting line is selected and the first package is separated from the strip by cutting along the cutting line. The cutting line can be selected near the seam so that the first package is opened upon cutting. The cutting line may alternatively be located on the seam, wherein the separated package remains closed. In this case, the package may, however, be partially opened by cutting a short opening near the seam.

The medication dispenser that is operated according to the method of the invention is capable of delivering packages to a patient one package at a time according to the information provided by labels of the packages. The medication dispenser may be configured to operate in such a manner that after the seam between the first package and the second package is recognised, the information on a label of the first package is determined. The first package is then separated from the strip and delivered to the patient so that the patient can take the medications at the predetermined taking time that is recognised from the label of the first package. Other packages of the strip may be processed in a similar manner.

The strip of packages is arranged in a container from where the packages of the strip are transferred to the imaging area of the camera. The strip may be inserted into the container by a patient or a caregiver of the patient, such as a nurse or a near relative. The strip comprises at least two medication packages, which are arranged in the strip sequentially in time order according to the taking time. Each package contains a dosage to be taken at a prescribed time. The packages are bags or pouches, which are preferably made of plastic.

The head of the strip may be transferred to the imaging area of the camera either manually by the patient or the caregiver, or automatically by transfer means of the medication dispenser. The transfer means are arranged to transfer packages from the container to the imaging area of the camera and to a cutter and then to an outlet of the medication dispenser.

With the present invention seams between consecutive packages in a strip can be detected accurately, and thus the packages can be cut from the strip at the correct places.

According to an embodiment of the invention, in the step of detecting the position of the seam, the strip is moved discontinuously and the images are captured when the strip is stationary. The strip may be moved in steps having a fixed length or in steps having differing lengths. For example, the length of the steps may be arranged to decrease as the seam recognition process proceeds. The length of the steps can be, for example, 1-5 mm, 5-10 mm, 10-30 mm, or 30-100 mm. The strip may also be moved in steps when transferring the head of the strip to the imaging area or moving the strip from one seam to another. In these cases long steps are usually used. On the other hand, short steps are needed for finetuning the seam to the cutter.

According to an embodiment of the invention the substeps of moving the strip, capturing at least one image and analysing the at least one image are repeated at most a predefined number of times. The substeps may be repeated, for example, at most 5-10 or 10-20 times to find the seam or a specific information. Typically, between these sets of steps imaging conditions and pattern recognition parameters can be changed. Imaging conditions and pattern recognition parameters include, for example, lighting and usage of specific digital filters and filter parameters. These changes may be done 1-5 times between the unsuccessful steps.

According to an embodiment of the invention the method comprises sending the images over a communications network to a server, if the position of the seam is undetected. At the server the images are analysed further by more sophisticated, resource demanding algorithms or manually by the service personnel. If the position of the seam can be detected, the position information of the seam is sent over the communications network to the medication dispenser.

According to an embodiment of the invention the method comprises giving a notification, if the position of the seam is undetected. The patient, the caregiver or any other authorised person may be notified if the position of the seam cannot be detected at the server, and/or in a situation where a seam cannot be detected after repeating the substeps of moving the strip, capturing at least one image and analysing the at least one image for a predefined number of times. The patient may be notified with an audio or visual signal so as to draw the attention of the patient to the fact that the seam cannot be detected. The patient may be notified, for example, with an alarm that is played using a loudspeaker, or visually using a lamp or a display. The caregiver or any other authorised person may be notified, for example, with a message sent over a communications network to a mobile device. The message can be, for example, an SMS (short message service) or MMS (multimedia messaging service) message.

According to an embodiment of the invention the method comprises capturing at least one image of a label of the package, combining the at least one image into a single image, analysing the image using pattern recognition to detect positions and formats of patterns in the image, selecting a layout from a set of layouts stored in the medication dispenser, which layout has similar pattern formats in the same positions as the image, the layout defining the type of information for each pattern in the image, and interpreting the information contained in at least one of the patterns of the image by linking the content of the pattern to the type of information defined in the layout. Preferably, the interpreted information contains the taking time of the medications in the package, so that the medication dispenser is able to dispense the medications to the patient at the correct time.

The method enables to recognise information on different types of labels, wherein the use of the medication dispenser is not limited to a specific label type.

A label of a package contains package-related information. The label comprises patterns, which have specific positions and formats, to which patterns the information is embedded. Patterns can have different formats, such as text, or a one- or two-dimensional bar code. Each pattern comprises a certain type of information. This information can be, for example, identification information of the person to whom the packages are meant to be dispensed, such as his/her name or social security number, and/or information related to the medical regimen of the person, such as the content of the package, and the taking time of the medications.

A layout can be specified for each label type. A layout defines the positions and formats of patterns for a certain label type. A layout also defines the type of information for each pattern in the layout.

According to an embodiment of the invention the method comprises sending the image over a communications network to a server, if a potential error in information recognition is detected. At the server the image is analysed further by more sophisticated, resource demanding algorithms or manually by the service personnel and information is corrected or approved at the server. The correct information is sent over the communications network to the medication dispenser.

If the set of layouts stored in the medication dispenser does not include a layout, which has similar pattern formats in the same positions as the image, a new layout can be generated. The generation of a new layout may be based on the recognised patterns in the image and information available in the medication dispenser and/or information fed to the medication dispenser during refilling. First, the printing patterns are analysed to find text and one- or two-dimensional bar code fields. These fields are interpreted to data strings. The data strings are further analysed based on the information in the medication dispenser. This information includes, for example, a patient name, a patient ID, current date and time, medication history and the taking time of the last package in the preceding strip. Based on this information, for example, the patient name, patient ID and medication taking time are searched from the data strings. If matching strings or parts of the strings are found, a preliminary layout is generated based on them and the next two packages are processed by using the preliminary layout. Fixed data, such as the patient name and patient ID, should be the same in each package. The detected taking times should be in consecutive order and should follow closely the medication history. The strip can only be changed during a refilling event. If the automatic layout generation does not succeed with available information, more information, such as the taking time of the first package and format of the date and time, can be asked from the refilling person. Based on this additional information the automatic layout generation is restarted. Finally, manual approval can be asked for the recognised information from the refilling person.

The new layout can be sent over a communications network to a server. At the server it can be analysed further and saved as a new layout in a layout database, so that it may be downloaded to other medication dispensers.

According to an embodiment of the invention the method comprises sending the images over a communications network to a server, if the generation of a new layout is unsuccessful. At the server the images are analysed further by more sophisticated, resource demanding algorithms or manually by the service personnel and a new layout is generated at the server. The new layout is sent over the communications network to the medication dispenser.

According to an embodiment of the invention the method comprises illuminating with at least one light source the part of the strip that is located in the imaging area. By illuminating the part of the strip that is located in the imaging area, the image quality can be increased. In many cases the use of at least two light sources is preferable because the light can then be directed to the strip from different directions. Preferably, the light sources are LEDs.

According to an embodiment of the invention the method comprises changing the intensity and/or the direction and/or the wavelength of the lighting. The intensity and/or the direction and/or the wavelength of the lighting may be changed between the capturing of images in order to increase the image quality.

Lighting from the sides or back of the package highlights the seam area from the other parts of the strip in the image to be processed. Typically, large contrast changes are formed on the area of the seam, which forms a certain pattern including a non-flat dotted perforation and blurry glued area. Especially the side lighting is useful for highlighting seam patterns in the strip. Lighting from different directions can be dynamically adjusted for different strip types to find an optimal lighting condition for a specific strip. The dynamical adjustment can be automatically run when the device has been refilled and potentially another strip type has been detected.

According to an embodiment of the invention the method comprises vibrating and/or straightening the part of the strip that is located in the imaging area. A purpose of the vibrating and/or straightening is to reorganise medication pills lying on each other so that a package becomes flat. Another purpose of the straightening is to keep the strip straight during imaging. Preferably, the package is vibrated and/or straightened before capturing each image.

According to an embodiment of the invention the method comprises changing the position of the camera. The position of the camera can be changed between the capturing of the images. A purpose of moving the camera is to enable scanning of the strip surface in a direction of the width of the strip. This enables taking several images of the uneven surface and combining them to enhance image quality for image processing. Preferably, several pictures are taken, if recognition faults are detected.

The invention also relates to a medication dispenser. A typical medication dispenser according to the invention comprises a container arranged to receive a strip of packages containing medications to be taken at predetermined taking times, each package having a label containing information relating to the package, a camera arranged to capture images of the strip, a cutter arranged to separate packages from the strip, transfer means for transferring packages from the container to an imaging area of the camera and to the cutter and then to an outlet of the medication dispenser, and a control unit arranged to control the camera, the cutter and the transfer means. In a typical medication dispenser according to the invention the control unit comprises means for analysing images using pattern recognition to detect the position of a seam between two consecutive packages of the strip.

The medication dispenser is arranged to dispense packages to provide the patient with the proper dosage of medications at a prescribed time. The time at which the packages are to be delivered to the patient is read from the labels of the packages. The medication dispenser allows the dispensation of medications to be monitored and controlled so that the patient, a caregiver of the patient or any other person having access to the medication dispenser can be assured that the patient is taking the medications as prescribed.

The control unit is connected to the camera, the cutter and the transfer means, and arranged to control the camera, the cutter and the transfer means so that one package is dispensed at a time at determined times. The packages are transferred with the transfer means from the container to the camera and further to the cutter and then to the outlet of the medication dispenser, from which outlet the patient can take the package containing the medications. The transfer means may comprise for example one or more rollers, which are driven by means of an electric motor. The electric motor is controlled by the control unit. The control unit comprises a processor that is programmed to carry out the functions that are needed to operate the medication dispenser. The control unit also comprises a memory for storing, for example, the patient information.

The package to be dispensed is separated from the strip by a cutter and then transferred to the outlet. The cutter is preferably arranged to open the package, whereby the medications can easily be taken out of the package. The outlet is preferably provided with a lid that may be lockable so that the access of the patient to the outlet can be prevented if desired.

According to an embodiment of the invention the control unit comprises means for analysing an image using pattern recognition to detect positions and formats of patterns in the image, means for maintaining a set of layouts in the medication dispenser, means for selecting a layout from the set of layouts, which layout has similar pattern formats in the same positions as the image, the layout defining the type of information for each pattern in the image, and means for interpreting the information contained in at least one of the patterns of the image by linking the content of the pattern to the type of information defined in the layout.

According to an embodiment of the invention the medication dispenser comprises at least one light source arranged to illuminate the part of the strip that is located in the imaging area.

According to an embodiment of the invention the medication dispenser comprises a detector arranged to detect the presence of a strip in the container. When inserting the strip, the strip is detected by means of the detector. The detector produces a signal according to which the control unit controls the transfer means to transfer the head of the strip to the imaging area of the camera.

According to an embodiment of the invention the medication dispenser comprises a communications unit arranged to communicate with a server over a communications network. The communications unit is capable of transmitting data to the server and receiving data from the server.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the method as well as the medication dispenser according to the invention, even though this is not always separately mentioned.

### BRIEF DESCRIPTION OF THE DRAWINGS

The novel features which are considered as characteristic of the invention are set forth in particular in the appended claims. The invention itself, however, both as to its construction and its method of operation, together with additional objects and advantages thereof, will be best understood from the following description of specific embodiments when read in connection with the accompanying drawings.
- Fig. 1: illustrates a medication dispenser according to an embodiment of the invention, and
- fig. 2: illustrates a schematic diagram of a method according to an embodiment of the invention.

### DETAILED DESCRIPTION OF THE DRAWINGS

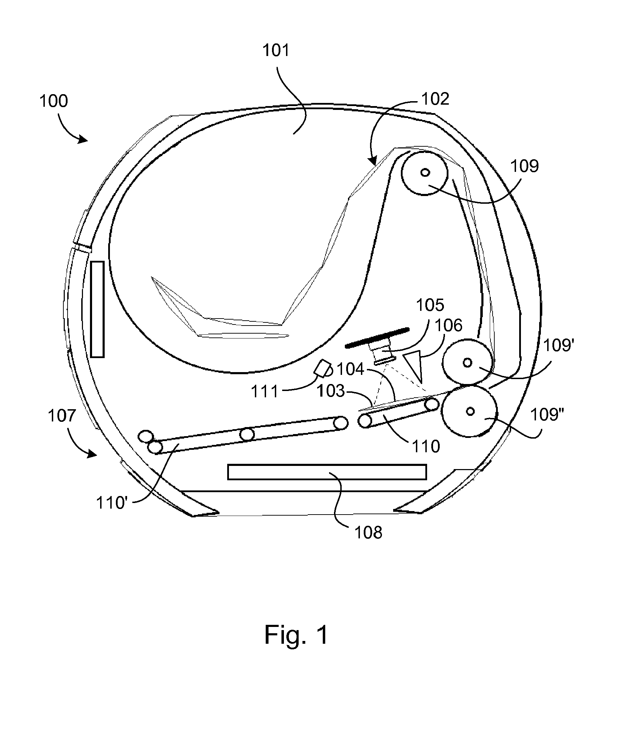

Fig. 1 illustrates a medication dispenser according to an embodiment of the invention. The medication dispenser 100 comprises a container 101, which is arranged to receive a strip 102 of packages 103 that include medications to be taken at predetermined taking times. Each package 103 has a label 104 that contains information relating to the package 103. The medication dispenser 100 also comprises a camera 105, which is arranged to capture images of the part of the strip 102 that is located in an imaging area of the camera 105, and a cutter 106 that is used for separating the packages 103 from the strip 102. The medication dispenser 100 further comprises transfer means for transferring the packages 103 of the strip 102 from the container 101 to the imaging area of the camera 105 and then to an outlet 107 of the medication dispenser 100, and a control unit 108 arranged to control the camera 105, the cutter 106 and the transfer means. The transfer means comprises rollers 109, 109', 109", which are arranged to transfer the strip 102 from the container 101 to the imaging area of the camera 105. The transfer means also comprises a roller table 110 that is arranged to transfer the strip across the imaging area, and a roller table 110' that is arranged to transfer the separated packages 103 to the outlet 107 of the medication dispenser 100. The medication dispenser 100 further comprises a light source 111 that is arranged to illuminate the part of the strip 102 that is located in the imaging area.

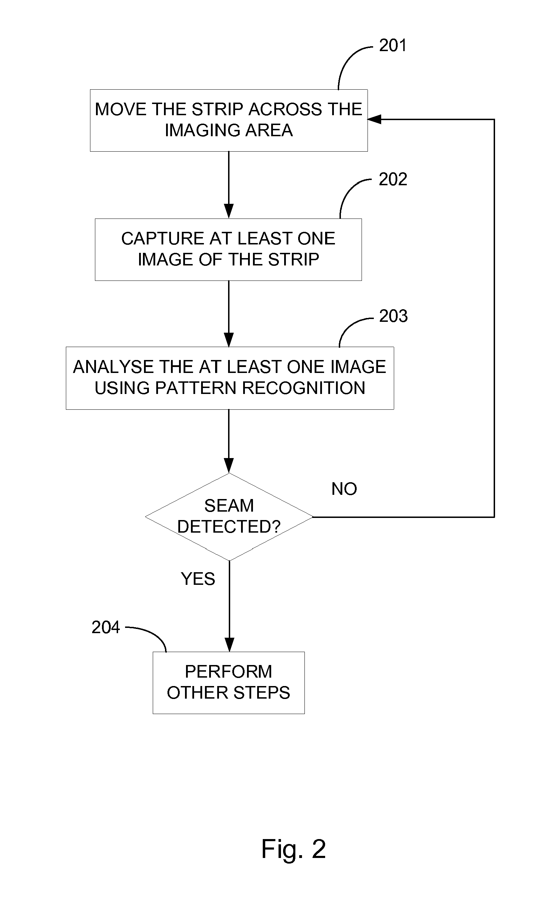

Fig. 2 illustrates a schematic diagram of a method according to an embodiment of the invention for detecting a seam between two consecutive packages in a strip.

At step 201 the strip 102 is moved across the imaging area of the camera 105. The strip 102 may be moved continuously or discontinuously across the imaging area.

At step 202 at least one image of the strip 102 is captured. Images are preferably captured of the strip 102 when the strip 102 is not moving, although images may also be taken while the strip 102 is being moved. Preferably, the camera 105 is positioned in such a manner that the whole width of the strip 102 fits in the imaging area and thus in a single image. If the strip 102 is wider than the imaging area, the camera 105 can be moved in a direction of the width of the strip 102 and a plurality of images can be captured and then combined.

At step 203 the at least one image is analysed using pattern recognition to detect the position of the seam. The images can be analysed separately, or the images can be combined to a single image in order to enhance the image quality.

If the position of the seam is detected, the method is continued with other operational steps at step 204. However, if the position of the seam cannot be detected, the procedure is continued at step 201.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A method for operating a medication dispenser that includes a strip of packages containing medications to be taken at predetermined taking times, **characterised in that** the method comprises:
- detecting the position of a seam between two consecutive packages in the strip, the step comprising:
- moving the strip across an imaging area of a camera,
- capturing at least one image of the strip,
- analysing the at least one image using pattern recognition to detect the position of the seam, and
- repeating the substeps of moving the strip, capturing at least one image and analysing the at least one image, until the position of the seam is detected.

2. The method according to claim 1, **characterised in that**, in the step of detecting the position of the seam, the strip is moved discontinuously and the images are captured when the strip is stationary.

3. The method according to claim 1 or 2, **characterised in that** the substeps of moving the strip, capturing at least one image and analysing the at least one image are repeated at most a predefined number of times.

4. The method according to claim 3, **characterised in that** the method comprises sending the images over a communications network to a server, if the position of the seam is undetected.

5. The method according to claim 3 or 4, **characterised in that** the method comprises giving a notification if the position of the seam is undetected.

6. The method according to any of the preceding claims, **characterised in that** the method comprises:
- capturing at least one image of a label of the package,
- combining the at least one image into a single image,
- analysing the image using pattern recognition to detect positions and formats of patterns in the image,
- selecting a layout from a set of layouts stored in the medication dispenser, which layout has similar pattern formats in the same positions as the image, the layout defining the type of information for each pattern in the image, and
- interpreting the information contained in at least one of the patterns of the image by linking the content of the pattern to the type of information defined in the layout.

7. The method according to any of the preceding claims, **characterised in that** the method comprises illuminating with at least one light source the part of the strip that is located in the imaging area.

8. The method according to claim 7, **characterised in that** the method comprises changing the intensity and/or the direction and/or the wavelength of the lighting.

9. The method according to any of the preceding claims, **characterised in that** the method comprises vibrating and/or straightening the part of the strip that is located in the imaging area.

10. The method according to any of the preceding claims, **characterised in that** the method comprises changing the position of the camera.

11. A medication dispenser, comprising:
- a container arranged to receive a strip of packages containing medications to be taken at predetermined taking times, each package having a label containing information relating to the package,
- a camera arranged to capture images of the strip,
- a cutter arranged to separate packages from the strip,
- transfer means for transferring packages from the container to an imaging area of the camera and to the cutter and then to an outlet of the medication dispenser, and
- a control unit arranged to control the camera, the cutter and the transfer
means;
**characterised in that** the control unit comprises means for analysing images using pattern recognition to detect the position of a seam between two consecutive packages of the strip.

12. The medication dispenser according to claim 11, **characterised in that** the control unit comprises:
- means for analysing an image using pattern recognition to detect positions and formats of patterns in the image,
- means for maintaining a set of layouts in the medication dispenser,
- means for selecting a layout from the set of layouts, which layout has similar pattern formats in the same positions as the image, the layout defining the type of information for each pattern in the image, and
- means for interpreting the information contained in at least one of the patterns of the image by linking the content of the pattern to the type of information defined in the layout.

13. The medication dispenser according to claim 11 or 12, **characterised in that** the medication dispenser comprises at least one light source arranged to illuminate the part of the strip that is located in the imaging area.

14. The medication dispenser according to any of claims 11 to 13, **characterised in that** the medication dispenser comprises a detector arranged to detect the presence of the strip in the container.
